# EUROPÄISCHE PATENTSCHRIFT

(11) **EP 1 412 480 B1**
(45) Veröffentlichungstag und Bekanntmachung des Hinweises auf die Patenterteilung: **25.06.2008**
(21) Anmeldenummer: 02762371.9
(22) Anmeldetag: 17.07.2002
(51) Int. Cl.: C12N 5/00

(54) **VERFAHREN ZUR VERHINDERUNG DER ADHÄSION VON PARTIKELN**
METHOD FOR PREVENTING THE ADHESION OF PARTICLES
PROCEDE POUR EMPECHER L'ADHESION DE PARTICULES

(30) Priorität: 27.07.2001 DE 10136722
(43) Veröffentlichungstag der Anmeldung: 28.04.2004
(73) Patentinhaber: PerkinElmer Cellular Technologies Germany GmbH, 22525 Hamburg (DE)
(72) Erfinder: GRADL, Gabriele, 13503 Berlin (DE)
(74) Vertreter: Hertz, Oliver
(86) Internationale Anmeldenummer: PCT/EP2002/007964
(87) Internationale Veröffentlichungsnummer: WO 2003/012077

(56) Entgegenhaltungen:
- WO-A-98/47948
- JP-A- 4 316 483
- US-A- 5 229 366
- US-A- 5 977 252
- HOLM P ET AL: "High bovine blastocyst development in a static in vitro production system using SOFaa medium supplemented with sodium citrate and myo-inositol with or without serum-proteins." THERIOGENOLOGY, Bd. 52, Nr. 4, 1999, Seiten 683-700, XP002223348 ISSN: 0093-691X
- GILGES ET AL: 'CZE Separations of Basic Proteins at Low pH in Fused Silica Capillaries with Surfaces Modified by Silane Derivatization and/or Absorption of Polar Polymers' HRC JOURNAL OF HIGH RESOLUTION Bd. 15, Nr. 7, 1992, Seiten 452 - 457, XP000371733
- GILGES ET AL: 'Capillary Zone Electrophoresis Separations of Basic and Acidic Proteins Using Poly(vinyl alcohol) Coatings in FUsed Silica Capillaries' ANAL.CHEM Bd. 66, 1994, Seiten 2038 - 2046, XP000996827
- ISHIKAWA M. ET AL: 'Glycerol attenuates the adherence of leukocytes in rat pial venules after transient middle cerebral artery occlusion.' NEUROLOGICAL RESEARCH Bd. 21, 1999, Seiten 785 - 790

## Beschreibung

Die Erfindung betrifft ein Verfahren zur Verhinderung der Adhäsion von Zellen an Oberflächen, wobei der Lösung, in der die Partikel enthalten sind, ein Polyvinylalkohol zugesetzt wird.

Das Anhaften an Oberflächen ist eine fundamentale Eigenschaft von Zellen. Dies zeigt sich beispielsweise in der Wechselwirkung von Zellen mit einer extrazellulären Matrix, ein wichtiger Faktor für die Kontrolle der Genexpression bzw. generellen Zellfunktion. Diese Prozesse werden insbesondere über Proteine an der Zelloberfläche beeinflußt.

Allerdings stellt diese Eigenschaft der Zellen bei der Herstellung von Zellkulturen und der zerstörungsfreien Untersuchung von Zellen oder Zellbestandteilen häufig ein großes Problem dar. Eine Anhaftung der Zellen beipielsweise an Oberflächen der zur Herstellung verwendeten Hilfsmittel und Gefässe ist hier unerwünscht. Insbesondere Technologien zur Mikroanalytik von biologischen Proben können nicht ohne Probleme für Zelluntersuchungen genutzt werden. Diese Systeme, die beispielsweise Kanalsysteme enthalten, wie in der "Lab-on-Chip"-Technologie, haben häufig nur Dimensionen von wenigen Mikrometern. Zudem ist die Auswahl der zum Einsatz kommenden Oberflächen-Materialien dieser Mikroanalysesysteme an den jeweiligen Herstellungsprozeß bzw. das Material der Mikrostrukturen an sich gebunden. Beispielsweise werden Mikrokananalysesysteme aus Glas, Silizium, Silikon und organischen Polymeren, wie PMMA oder Polyurethan, hergestellt; alles Substrate, die eine Adhäsion von Zellen begünstigen. Das Oberflächenmaterial kann somit nicht frei nach den Erfordernissen für eine minimale Zelladhäsion gewählt werden.

Zur Vermeidung von Zellanhaftung/Adhäsion wird daher die Oberfläche der Mikrostrukturen selbst modifiziert. Dazu werden verschiedene chemische Verbindungen, i. A. hydrophobe Silane und hydrophile Polymere (Hydrogele) eingesetzt.

Carlson et al. ((1997): Self-Sorting of White Blood Cells in a Lattice. Physical Review letters 79 Nr. 11, S. 2149-2152) gelang es, mit den bekannten Beschichtungen Blutzellen beispielsweise in mechanischen Mikrofiltersystemen zu sortieren. Allerdings blieben dabei weiße Blutkörperchen irreversibel an den Eingängen von Mikrokanälen haften. Dies ist in dem beschriebenen System erwünscht, zeigt aber zugleich die derzeitigen Einschränkungen im Einsatz von Mikrokanälen für die zerstörungsfreie Untersuchung zellulärer Proben.

Weitere Untersuchungen von Li et al. (Transport, Manipulation, and Reaction of Bological Cells On-Chip Using Electrokinetic Effects. Analytical Chemistry (1997) 69, 1564-1568) zeigten, dass Blutzellen in 40 µm breite Mikrokanäle eingebracht werden können. Allerdings wurden die Zellen anschließend mit einem Reagenz lysiert und erst dann einer Analyse unterzogen. Die Wiedergewinnung aber von eukaryotischen, insbesondere von lebenden Säugerzellen, aus Mikrokanälen stellt hohe Ansprüche an die Auswahl der Oberflächen-Materialien der Kanäle.

Weitere Untersuchungen, beispielweise die Beschichtung von Glasplättchen mit Polyvinylalkohol, zeigten, dass eine solche Oberflächenmodifikation eine Adhäsion von Mausfibroblasten minimieren kann (Hisada, T. (1976): The adhesion of culture cells to some polymers (japanisch). Shika Rikogaku Zasshi 17(38), S. 91-101)).

Ebenfalls konnte gezeigt werden, dass Co-Polymerpartikel aus Poly(methylmethacrylat) und Poly(vinylalkohol) nicht an Blutzellen adhärieren und von Monozyten und Neutrophilen nicht phagozytiert werden (Ayhan, H. und E. Piskin (1997): Interaction of activated leukocytes with polymeric microspheres. International Journal of Artificial Organs 20(12), 704-707).

Krylov et al. (Electrophoresis, 21, 767-773, 2000) stellten zudem fest, dass sich die Haftung von Zellen an Glas oder Polystyrol deutlich verringert, wenn die Oberflächen mit hydrophilen Polymeren (Hydrogelen), wie z.B. Polyvinylalkohol modifiziert werden.

Allerdings ist es bei allen genannten Untersuchungen notwendig, eine direkte Oberflächenbeschichtung durchzuführen, wobei beispielsweise Polyvinylkohol (PVA) immobilisiert auf der Oberfläche gebunden ist, d. h. eine Oberflächenbeschichtung oder eine Copolymerisation eines Trägers mit PVA durchgeführt wird. Zur Durchführung der Beschichtung sind komplizierte, insbesondere an genaue Temperaturvorgaben gebundene, mehrstufige und dadurch sehr zeitintensive Prozesse notwendig. Darüber hinaus ist insbesondere die Beschichtung von Oberflächen, die aus Polymeren, wie z. B. Polystyrol, bestehen, problematisch, da dieses Material durch die zur Beschichtung notwendigen Temperaturen von weit über 100°C beschädigt werden kann. Das Verfahren kann somit zufriedenstellend nur für die Verwendung mit Glasoberflächen eingesetzt werden.

Weiterhin ist es oft unmöglich, insbesondere aufgrund der nur geringen Durchmesser der Mikrostrukturen in Mikroanalysesystemen, eine zufriedenstellende Beschichtung zu erzielen. So ist beispielsweise eine gleichmäßige räumliche Verteilung der Polymere oder anderen Beschichtungsmaterialien über die Oberflächen der entsprechenden Mikrostrukturen, beispielsweise von Kanalstrukturen von nur wenigen Mikrometern Durchmesser, nicht oder nur unzureichend möglich.

Ishikawa et al., Neurological Research, Bd. 21, 1999, Seiten 785-790, offenbaren die Verwendung von Glycerin zur Vermeidung bzw. Verringerung der Adhäsion von Leukozyten an Endothelzellen des Gehirns *in vivo*, nicht in *in vitro.*

Gilges et al. beschreiben in zwei Veröffentlichungen, HRC Journal of High Resolution, Bd. 15, Nr. 7, 1992, Seiten 452-457, und Anal. Chem. Bd. 66, 1994,Seiten 2038-2046, jeweils Verfahren zur Verhinderung der Adhäsion von elektrophoretisch aufgetrennten Proteinen an Siliciumkapillaren durch die Zugabe von Polvinylalkohol zum sauren Laufpuffer. Diese Verfahren sind nicht zur Verhinderung der Adhäsion von intakten Zellen an Oberflächen geeignet.

Daher ist es Aufgabe der vorliegenden Erfindung, ein Verfahren zu entwickeln, das unabhängig von der Art und den OberflächenEigenschaften eines Mikroanalysesystems eine im wesentlichen zerstörungsfreie und verlustfreie Untersuchung von Partikeln, insbesondere von Zellen und Zellbestandteilen, in Lösung in diesen Systemen ermöglicht.

Die Aufgabe wird gelöst durch ein Verfahren nach Anspruch 1 und die eine verwendung Lösung nach Anspruch 14.

Das Verfahren umfasst erfindungsgemäß die Zugabe einer Substanz zu der Lösung, die die Zellen enthält. Diese erfindungsgemäße Substanz bewirkt, das die Anhaftung dieser Zellen an Oberflächen, insbesondere Oberflächen eines Mikroanalysesystems im wesentlichen verhindert wird.

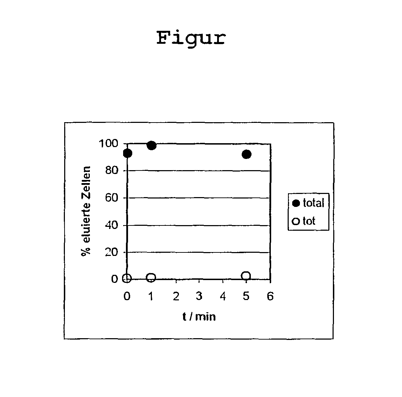

In der einzigen Figur wird ein Diagramm gezeigt, das die Wiedergewinnungsrate von suspendierten Jurkat-Zellen aus einer Mikroliter-Spritze in Abhängigkeit von der Verweildauer t in Gegenwart von 0,5% PVA erläutert.

Erfindungsgemäß handelt es sich bei dieser Substanz um einen Polyvinylalkohol, beispielsweise Polyvinylalkohol VA 30000-70000. Überraschenderweise hat die Zugabe dieser Substanz zur Lösung keinen toxischen Effekt in Bezug auf lebende Zellen gezeigt oder bewirkt keine Veränderung von Zellbestandteilen. Besonders geeignet ist eine Konzentration des Polyvinylalkohols in Lösung von 0.01% (w/v) bis 5% (w/v). Es konnte sogar gezeigt werden, dass die Anti-Adhäsionswirkung des Polyvinylalkohols die von bekannten als Anti-adhäsiva eingesetzten Substanzen, weit übertrifft (Tabelle 1). Die Wasserlöslichkeit und weitere im folgenden aufgeführten Eigenschaften des Polyvinylalkohols bewirken eine universelle Anwendbarkeit in allen wäßrigen Partikellösungen.

Eine weitere wichtige Eigenschaft dieser Substanz, die sie insbesondere für den Einsatz in Lösungen, die Zellen und/oder Zellbestandteile enthalten, qualifiziert, ist, dass sie keinen Einfluß auf den physiologischen pH-Wert der Lösung hat.

Viele Analysenverfahren, die in Mikroanalysesystemen Anwendung finden, beruhen auf der Aufnahme und Auswertung optischer Signale, wie beispielsweise die Fluoreszenz-Korrelationsspektroskopie, Fluoreszenzresonanz-Energietransfer, Fluoreszenzpolarisation, Fluorescence Intensitiy Distribution Analysis, oder beispielsweise UV-Spektroskopie. Daher war es umso wichtiger zur universellen Einsetzbarkeit dieser Substanz, dass sie eine optische Analyse nicht beeinflußt, d.h. insbesondere keine Autofluoreszenz zeigt oder den Brechungsindex der Lösung verändert. Diese Anforderungen werden durch Polyvinylalkohole sehr gut erfüllt.

Das erfindungsgemäße Verfahren erlaubt nun eine im wesentlichen verlustfreie Untersuchung und Manipulierung, insbesondere Separation der Partikel, insbesondere in Mikroanalysesystemen.

Hierbei kann es sich um Mikrokanalysesysteme handeln, die beispielsweise Kanäle mit einer Höhe von 1 - 1000 µm, insbesondere 2-500µm, besonders bevorzugt 40 µm; einer Breite von 100 - 2000 µm, insbesondere 200 - 600 µm und einer Länge von 100 µm - 10 cm, bevorzugt 1-2 cm, aufweisen. Die Systeme können insbesondere aus Glas, Silizium, Silikon, organischen Polymeren oder einem anderen geeigneten Werkstoff bestehen. Besondere Ausführungsformen der Mikrokanalysesysteme können auch Elektroden insbesondere zur Erzeugung dielektrischer Kräfte zum Manipulieren der zu untersuchenden Teilchen, beispielsweise zum mikroskopischen, optischen und / oder elektrischen Analysieren, zum Sortieren, zum Separieren, zum Elektroporieren, zum Fusionieren und Trennen der Teilchen, insbesondere von Zellen, aufweisen.

So enthalten beispielsweise Mikrokanalysesysteme Elektroden als dielektrische Weichen in den Mikrokanälen zur Sortierung von suspendierten Partikeln (insbesondere Zellen) oder in anderen Mikrokanalysesysteme sind die Elektroden so angeordnet, dass Zellfusion und/oder Zellporation durchgeführt werden kann und andere Syteme enthalten zwei Elektrodenebenen in einem geschlossenen Mikrokanal, einen dielektrischen Feldkäfig in einer Kanalkreuzung sowie als dielektrische Weichen ausgebildete Elektroden zur Durchführung einer Stop-flow Analyse sowie einer nachfolgenden Sortierung der analysierten Partikel. Wobei Mikrokanalysesysteme auch Kombinationen verschiedener Elektrodensysteme enthalten können.

Selbst das Einspritzen der Partikel in ein solches System, was häufig mit Hilfe einer Kunststoff- oder Glas-Spritze durchgeführt wird, führt zu keinen Verlusten mehr, die durch Anhaften der Partikel an den Spritzenkörper verursacht werden.

Da die erfindungsgemäß zugegebene Substanz auch eine lebende Zelle nicht beeinflusst, ist es beispielsweise möglich, Zellen direkt zu untersuchen, aufgrund der Untersuchungen zu sortieren und dann gezielt mit den separierten Zellen erneut Zellkulturen anzulegen. So lassen sich z. B. Zellen mit einer bestimmten Eigenschaft, beispielsweise einer bestimmten Sorte Rezeptoren auf der Zelloberfläche, gezielt und im wesentlichen verlustfrei von anderen Zellen trennen und vermehren.

Das erfindungsgemäße Verfahren eignet sich generell für alle Zelltypen somit sowohl für eukaryotische Zellen als auch für prokaryotische Zellen.

In Tabelle 1 ist der Anteil lebender Zellen nach der Untersuchung und/oder Manipulation in verschiedenen Mikroanalysesystemen in Gegenwart von Polyvinylalkohol dargestellt. (Jurkat - Zellen in Cytocon™ Sorter Chips (Nr. 1 und 2), in Cytocon™ Porator Chips (Nr. 3-5) und Cytocon™ Loader Chips (Nr. 6 und 7)).

**Tabelle 1**

| | Flußrate in µl/h | | Zellmenge | | Wiedergewinnungsrate |
|---|---|---|---|---|---|
| Nr. | Probe | Hüllstrom | pro µl | gesamt | % |
| 1 | 10 | 288 | 660 | 440 | 84 |
| 2 | 10 | 288 | 132 | 220 | 64 |
| 3 | 10 | 288 | | 689 | 74 |
| 4 | 10 | 144 | | 144 | 100 |
| 5 | 10 | 144 | | 144 | 113 |
| 6 | 18 | 144 | 160 | 160 | 87,3 |
| 7 | 20 | 18 | 305 | 243 | 63 |

In Tabelle 2 ist jeweils der Anteil an Jurkat-Zellen und Blutzellen in den Fraktionen 1 und 2 nach einer Zellseparation in einem Mikroanalysesystem (Cytocon™ Chip) dargestellt.

**Tabelle 2**

| Anzahl Zellen Gesamt | | | Fraktion 1 | | | Fraktion 2 | | |
|---|---|---|---|---|---|---|---|---|
| total | Jurkat | RBC | total | Jurkat | RBC | total | Jurkat | RBC |
| 6355 | 161 | 6194 | 345 | 151 | 194 | 6010 | 10 | 6000 |
| 100% | 2.5% | 97.5% | 100% | 43.8% | 66.2% | 100% | 0.17% | 99.8% |

Das erfindungsgemäße Verfahren verhindert eine Anhaftung von Zellen an Oberflächen von Mikroanalysesystemen, die insbesondere aus Silizium, Silikon, Glas und/oder organischen Polymeren wie PMMA, Polyurethan, Polycarbonat, Polypropylen, Polystyrol, Polyethylen, Polyvinylchlorid, Teflon®, Polyacryl, Nylon® und/oder Perlon® bestehen können.

In Tabelle 3 ist die Zellanhaftung an Polystyrol-Kulturschalen in Gegenwart von verschiedenen Substanzen dargestellt.

**Tabelle 3**

| | *Jurkat* | | *U937* | |
|---|---|---|---|---|
| | Anhaftung | Vitalität | Anhaftung | Vitalität |
| RPMI 10% FBS | - | 97% | 9 | 100% |
| PBS | 999 | 99% | 999 | 98% |
| PBS + Ca, Mg | 999 | OK | 999 | OK |
| 0.1% BSA | 99 | OK | 999 | OK |
| 0.5% BSA | - | OK | 99 | OK |
| 1% PVP 15 | 99 | 100% | 999 | 100% |
| 1% PVP 25 | 99 | 1003 % | 999 | 100% |
| 1% PEG 5000 | 99 | 100% | 999 | 100% |
| 1% MC 15 | 99 | OK | 999 | OK |
| 1% MC 400 | 99 | OK | 99 | OK |
| 10% Ficoll 70 | 999 | OK | 999 | OK |
| 10% Ficoll 400 | 999 | OK | 999 | OK |
| 10% Dextran T10 | 994 | OK | 999 | OK |
| 1% PVA 30000-70000 | - | 97% | (9) | 98% |
| 0.1% PVA 30000-70000 | - | OK | (9) | OK |
| 0.01% PVA 30000-70000 | - | OK | (9) | OK |

| | | | | |
|---|---|---|---|---|
| Bemerkungen: - = Keine Anhaftung (9) = Sehr geringe Anhaftung 9 = Deutliche Anhaftung 99 = Starke Anhaftung 999 = Sehr starke Anhaftung OK = keine Quantifizierung, aber kein Unterschied zur Kontrolle erkennbar | | | | |

Wobei im Sinne dieser Erfindung mit Oberfläche jede Oberfläche gemeint ist, insbesondere aber Kanaloberflächen, Oberflächen von Vorratsgefäßen von Mikroanalysesystemen, Schläuchen, Spritzen, Injektionsmodulen oder Oberflächen synthetischer Mikropartikel.

Es wurde zudem eine Lösung gefunden, die generell für die Untersuchung von Partikeln, insbesondere von Zellen und Zellbestandteilen in Mikroanalysesystemen geeignet ist. Diese Lösung enthält salinen Puffer, insbesondere PBS, und Inositol und Polyvinylalkohol. Der Lösung können aber auch andere im Zusammenhang mit Zellösungen bekannte Bestandteile hinzugefügt werden, wie beispielsweise Magnesium- oder Calciumionen.

Die Anteile an salinem Puffer, Inositol und/oder PVA sind variabel. Der Puffer kann durch normoosmolare, hyperosmolare oder hypoosmolare Zuckerlösung (z. B. Sucrose oder Inositol) in variablem Mischungsverhältnis verdünnt sein. Der Anteil an salinem Puffer an der Lösung kann zwischen 0 und nahezu 100% variiert werden, wobei Anteile von 20%, 50 % oder nahezu 100% besonders bevorzugt sind.

Inositol wird bevorzugt als 0.2 bis 0.5 M Lösung, beispielsweise von Inositol in destilliertem Wasser, besonders bevorzugt 0.3 M Lösung verwendet. Wobei der Anteil an Inositol-Lösung und der erfindungsgemäßen Lösung ebenfalls zwischen 0 und nahezu 100% variiert werden kann. Besonders bevorzugt sind allerdings Anteile von 50%, 80% und nahezu 100%.

Für die erfindungsgemäße Lösung ist Polyvinylalkohol mit einem Molekulargewicht zwischen 30000 g/mol und 70000 g/mol besonders gut geeignet, wobei der Gehalt an PVA in der erfindungsgemäßen Lösung zwischen 0.1% (w/v) und 1.1% (w/v), bevorzugt zwischen 0.3%(w/v) und 0.6% (w/v), besonders bevorzugt zwischen 0.45% (w/v) und 0.55% (w/v) liegt.

Die erfindungsgemäße Lösung kann unabhängig von Art und Eigenschaften der Partikel, insbesondere für ihre Untersuchung und Manipulation in Mikroanalysesystemen eingesetzt werden.

Hierzu können die Partikel direkt in dieser Lösung suspendiert und in das Mikroanalysesystem eingebracht werden. Es ist aber auch möglich, diese Lösungen zu Partikelsuspensionen hinzuzufügen.

### Beispiel 1

### Einfluß von Polyvinylalkohol auf den pH-Wert einer Lösung

Der pH-Wert einer 1%igen Lösung von Polyvinylalkohol in PBS wurde gemessen und liegt bei 7.31 (physiologischer pH-Bereich ist pH 7.2 - 7.4).

### Beispiel 2

### Der Einfluß von Polyvinylalkohol auf die Zelladhäsion im Vergleich zu anderen Additiven

### Lösungen:

*Jurkat-*Zellen (Klon E6.1 von der European Collection of Animal Cell Cultures, Salisbury, England) und U937-Zellen (Monozytische Zellinie von der European Collection of Animal Cell Cultures, Salisbury, England) wurden in RPMI 1640 Medium (GIBCO Life Technologies, Karlsruhe) unter Zusatz von je 100 IU/ml Penicillin und Streptomycin (Seromed/Biochrom, Berlin) und 10% fötalem Kälberserum (Seromed/Biochrom, Berlin) kultiviert. Je 3 ml der Zellsuspension wurden abzentrifugiert und in 300 µl Phosphat-gepufferter Salzlösung ohne Calcium und Magnesium (PBS, Seromed/Biochrom, Berlin) aufgenommen. Die Wells einer 24-Well Platte (24-Well Polystryrol-Kulturschalen, Corning Costar) wurden mit je 500 µl PBS unter Zusatz der in Tabelle 1 bezeichneten Konzentrationen der nachfolgend aufgeführten Stoffe befüllt. Getestet wurden Methylcellulose (15 und 4000 centipoise Viskosität einer 2% igen Lösung, SIGMA Aldrich GmbH, Steinheim), Ficoll 70 und Ficoll 400 (Pharmacia, Uppsala, Schweden), Dextran T10 (Pharmacia, Uppsala, Schweden), Polyvinylpyrrolidon 15 und 25 (Serva), Polyethylenglycol 5000 (Fluka) und Polyvinylalkohol 30000-70000 (SIGMA Aldrich GmbH, Steinheim). Es wurden 50 µl der oben beschriebenen Zellsuspension zugegeben. Als Kontrolle diente eine Probe in 500 µl Zellkulturmedium (in dem keine Anhaftung dieser Suspensionszellen erfolgt).

### Durchführung:

Die Proben wurden 30 Minuten bei 37°C inkubiert. Der Überstand mit nicht-angehafteten Zellen wurde abgenommen und durch Puffer ersetzt. Die Anhaftung wurde qualitativ bewertet (siehe Tabelle 1). Die Zellen sowohl im Überstand als auch in den Wells wurden mit Trypanblaulösung (0,2%, SIGMA Aldrich GmbH, Steinheim) gefärbt, um die Vitalität der Zellen zu untersuchen. Lediglich Polyvinylalkohol hat einen sehr deutlich hemmenden Effekt auf die Zellanhaftung bei beiden Zellinien. Der Effekt tritt schon bei einer Konzentration von 0,1% (w/v) auf.

### Ergebnis:

Viele Substanzen, denen eine anti-adhäsive Wirkung zugesprochen wird, wurden im Vergleich zu Polyvinylalkohol getestet. Wie beispielsweise das aus der Durchflußzytometrie zum Blocken unspezifischer Bindungen bekannte und bei immunreaktiven Nachweisen (z. B. Western-Blot Technik) eingesetzte Rinderserumalbumin (BSA). Darüber hinaus wurden verschiedene hydrophile Polymere, die in der Dichtegradientenzentrifugation von Blutproben oder in Zellkulturtechniken zum Einsatz kommen, untersucht. Es wurde sogar der Einfluss von Cadherinen und Integrinen, spezifischen Zell-Zell- und Zell-Substrat-Adhäsionsmolekülen, in Puffern ohne Calcium und Magnesium untersucht. Diese Moleküle benötigen zur Interaktion Calcium. Es zeigte sich, das Polyvinylalkohol die weiteren Substanzen in der Wirkung erfolgreich eine Adhäsion suspendierter Zellen an den Probenträger zu verhindern, deutlich übertrifft.

### Beispiel 3

### Untersuchung der Toxizität von Polyvinylalkohol in Bezug auf lebende Säugerzellen

### Durchführung:

Die Verwendung von 0,5% (w/v) PVA für die Wiedergewinnung von Zellen aus einer Mikroliterspritze wurde untersucht. Jurkat-Zellen wurden in einer Konzentration von 5,69 x 10⁵ Zellen pro ml in PBS mit 0,5% PVA (w/v) suspendiert. 5 µl dieser Suspension wurden in eine 5 µl-Spritze (Dynatech) aufgezogen. Je 1 µl wurde sofort und nach 1 bzw. 5 Minuten in ein Well einer Terasaki-Platte (NUNC, Wiesbaden) abgegeben. Die Zellen wurden in der Platte mit dem Live/Dead Stain Kit (Molecular Probes, Leiden, Niederlande) angefärbt und gezählt, dabei wurde gleichzeitig ihre Vitalität untersucht. Die Wiedergewinnungsrate wurde ermittelt.

### Ergebnis:

Über 90% der Zellen konnten - auch nach 5 Minuten - aus der Spritze wiedergewonnen werden. Sie hatten sich folglich nicht angehaftet. Der Prozentsatz an toten Zellen betrug zu Beginn, also bei 0 Minuten 0,8% und stieg nach 5 Minuten nur auf 2,4%.

### Beispiel 4

### Untersuchung von Zellen in Mikroanalysesystemen unter Verwendung eines Zellpuffers mit Polyvinylalkohol

### Material:

Der Einsatz von Polyvinylalkohol für die Arbeit mit suspendierten Zellen in Mikrokanälen wurde mit einem Cytocon™ 300 Gerätesystem untersucht. Die Cytocon™ Chips bestehen aus Glas und weisen Kanäle mit einer Höhe von 40 µm, einer Breite von 200 - 600 µm und einer Länge von ca. 1-2 cm auf, wobei spezielle Ausführungsformen der Chips auch Elektroden in den Kanälen aufweisen können. Cytocon™ Sorter Chips enthalten beipielsweise Elektroden als dieelektrische Weichen in den Mikrokanälen zur Sortierung der supendierten Partikel (insbesondere Zellen), in Cytocon™ Porator Chips sind die Elektroden so angeordnet, dass Zellfusion und/oder Zellporation durchgeführt werden kann und in Cytocon™ Loader Chips, die zwei Elektrodenebenen in einem geschlossenen Mikrokanal, einen dielektrischen Feldkäfig in einer Kanalkreuzung sowie als dielektrische Weichen ausgebildete Elektroden aufweisen, sind zur Durchführung einer Stop-flow Analyse sowie der nachfolgenden Sortierung der analysierten Partikel geeignet.

Polyvinylalkohol mit einem Molekulargewicht von 30000 g/mol bis 70000 g/mol wurde eingesetzt. Der Polyvinylalkohol wurde im Puffer zu 0,5% (w/v) gelöst und war während der gesamten Bearbeitung der Zellen in den Mikrokanälen anwesend.

### Durchführung:

Die Zellen wurden in dem Puffer suspendiert und in einen Cytocon™ Chip injiziert. Die Suspension wurde innerhalb des Kanalsystems mit einer bestimmten Flußrate ("Probe") befördert und am Chipausgang mit einem Hüllstrom beschleunigt. Die ausgespülten Zellen wurden gezählt und aus der Ausgangskonzentration und der eingespritzen Probenmenge die Wiedergewinnungsrate ermittelt.

### Ergebnis

Obwohl die eingesetzte Zellmenge nur bei wenigen hundert Zellen lag und die Flußrate in den Mikrokanälen sehr langsam war (Aufenthaltsdauer der Zellen im Mikrokanal ca. 2 Minuten), war die Wiedergewinnungsrate an Zellen sehr hoch (Tabelle 2) und Werte um 100% waren zu erreichen.

### Beispiel 5

### Fraktionierung von Blutzellen (Trennung der Lyphozyten von roten Blutkörperchen)

### Durchführung:

Es wurde eine Mischung von menschlichem Vollblut, Jurkat-T-Lymphomzellen und PVA-haltigem Puffer hergestellt. Der Puffer enthielt 20% (V/V) PBS, 80% (V/V) 0,3 M Inositlösung und 0,5% (w/V) PVA (30.000 - 70.000, Sigma). Zur besseren Visualisierung während des Experimentes wurden Jurkat-Zellen mit 10µM Calcein-AM™ (Molecular Probes) markiert.

### Das Blut und die Lymphomzellen wurden wie folgt verdünnt:

Die Zellproben wurden zu Konzentrationen von 2.5·10⁶ Jurkat Zellen/ml und 1.8·10⁷ roten Blutkörperchen/ml gemischt. Proben von 0.3µl wurden in einen Cytocon™ Chip (Mikroanalysesystem) injiziert.

Die für diesen Versuch verwendeten Cytocon™ Chips bestanden aus Glas und wiesen Kanäle mit einer Höhe von 40 µm, einer Breite von 200 - 600 µm und einer Länge von ca. 1-2 cm auf. Zudem enthielt das hier verwendete Mikroanalysesystem in einem oder mehreren Mikrokanälen Elektroden, die hier als dielektrische Weiche zum Sortieren der Zellen angeordnet sind.

Die Zellen wurden basierend auf Unterschieden in ihrer Größe und ihren dieelektrischen Eigenschaften über die sog. Switch Elektrode (Weiche) des Cytocon™ Mikroanalysesystems bei einer Frequenz des elektrischen Feldes von 800kHz und einer Amplitude von 3-6V rms bei einer Flussrate von 62 - 240 µm/sec getrennt. Unter diesen Bedingungen wurden die Jurkat Zellen abgelenkt und in Fraktion 1 gesammelt, während die roten Blutkörperchen (RBC) die Weiche passieren konnten und in Fraktion 2 gesammelt wurden.

Jurkat Zellen und rote Blutkörperchen (RBC) wurden in beiden Fraktionen gezählt, um die Anreicherung und die Ausbeute bestimmen zu können.

### Ergebnis:

Der Anreicherungsfaktor für die Jurkat Zellen in der Fraktion 1 ist in einem Beispielexperiment 17.5. Die Ausbeute an Jurkat-Zellen in dieser Fraktion war in diesem Fall 93% (Tabelle 3) .

Insgesamt (Fraktionen 1 und 2) betrug die Zellwiedergewinnungsrate nahezu 100%.

## Patentansprüche

1. Verfahren zur Verhinderung der Adhäsion von Zellen an Oberflächen, **dadurch gekennzeichnet, dass** die Zellen in einer Lösung bereitgestellt werden, die einen salinen Puffer und Polyvinylalkohol enthält.

2. Verfahren nach Anspruch 1, **dadurch gekennzeichnet, dass** die Konzentration des Polyvinylalkohols in Lösung 0.01% (w/v) bis 5% (w/v) beträgt.

3. Verfahren nach Anspruch 2, **dadurch gekennzeichnet, dass** die Konzentration des Polyvinylalkohols in Lösung 0.01% (w/v) bis 1.1% (w/v) beträgt.

4. Verfahren nach mindestens einem der Ansprüche 1 bis 3, **dadurch gekennzeichnet, dass** die Zellen eukaryotische und/oder prokaryotische Zellen sind.

5. Verfahren nach mindestens einem der Ansprüche 1 bis 4, **dadurch gekennzeichnet, dass** es sich bei den Oberflächen um Zellen, Zellmembranen, Zellbestandteile, unbeschichtete oder mit Biomolekülen beschichtete Oberflächen aus Silizium, Silikon, organischen Polymeren und/oder Glas handelt.

6. Verfahren nach Anspruch 5, **dadurch gekennzeichnet, dass** die Biomoleküle Lipidmembranen, Lipide, Proteine (insbesondere Antikörper), DNS, Nukleinsäuren, Botenstoffe, Biotin, Zucker, Glykane und/oder Glykoproteine sind.

7. Verfahren nach Anspruch 5, **dadurch gekennzeichnet, dass** die organischen Polymere PMMA, Polyurethan, Polycarbonat, Polypropylen, Polystyrol, Polyethylen, Polyvinylchlorid, Teflon®, Polyacryl, Nylon® und/oder Perlon® sind.

8. Verfahren nach mindestens einem der Ansprüche 1 bis 7, **dadurch gekennzeichnet, dass** es sich bei den Oberflächen um Kanaloberflächen eines Mikroanalysesystems handelt.

9. Verfahren nach mindestens einem der Ansprüche 1 bis 7, **dadurch gekennzeichnet, dass** es sich bei den Oberflächen um Oberflächen synthetischer Mikropartikel handelt.

10. Verfahren nach mindestens einem der Ansprüche 1 bis 9, **dadurch gekennzeichnet, dass** die Zellen in einer Lösung bereitgestellt werden, welche enthält:
- salinen Puffer
- Inositol und
- Polyvinylalkohol.

11. Verfahren nach Anspruch 10, **dadurch gekennzeichnet, dass** es sich bei dem salinen Puffer um PBS handelt.

12. Verfahren nach Anspruch 10 oder 11, **dadurch gekennzeichnet, dass** der Anteil an salinem Puffer und/oder Inositol variabel ist.

13. Verfahren nach mindestens einem der Ansprüche 10 bis 12, **dadurch gekennzeichnet, dass** der Gehalt an PVA zwischen 0.1% (w/v) und 1.1% (w/v), bevorzugt zwischen 0.3%(w/v) und 0.6% (w/v), besonders bevorzugt zwischen 0.45% (w/v) und 0.55% (w/v) liegt.

14. Verwendung einer Lösung, enthaltend:
- salinen Puffer
- Inositol, und
- Polyvinylalkohol,
wobei der Gehalt an Polyvinylalkohol zwischen 0.1% (w/v) und 1.1% (w/v) liegt,
zur Verhinderung der Adhäsion von Zellen an Oberflächen in Mikroanalysesystemen.

15. Verwendung nach Anspruch 14, **dadurch gekennzeichnet, dass** es sich bei dem salinen Puffer um PBS handelt.

16. Verwendung nach Anspruch 14 oder 15, **dadurch gekennzeichnet, dass** der Gehalt an Polyvinylalkohol zwischen 0.3%(w/v) und 0.6% (w/v), bevorzugt zwischen 0.45% (w/v) und 0.55% (w/v) liegt.

## Claims

1. Method for preventing the adhesion of cells on surfaces, **characterised in that** the cells are provided in a solution which contains a saline buffer and polyvinyl alcohol.

2. Method according to claim 1, **characterised in that** the concentration of the polyvinyl alcohol in solution is 0.01% (w/v) to 5% (w/v).

3. Method according to claim 2, **characterised in that** the concentration of the polyvinyl alcohol in solution is 0.01% (w/v) to 1.1% (w/v).

4. Method according at least to one of the claims 1 to 3, **characterised in that** the cells are eukaryotic and/or prokaryotic cells.

5. Method according at least to one of the claims 1 to 4, **characterised in that** the surfaces are cells, cell membranes, cell components, surfaces made of silicon, silicone, organic polymers and/or glass which are uncoated or coated with biomolecules.

6. Method according to claim 5, **characterised in that** the biomolecules are lipid membranes, lipids, proteins (in particular antibodies), DNA, nucleic acids, messenger chemicals, biotin, sugar, glycans and/or glycoproteins.

7. Method according to claim 5, **characterised in that** the organic polymers are PMMA, polyurethane, polycarbonate, polypropylene, polystyrene, polyethylene, polyvinyl chloride, Teflon®, polyacryl, Nylon® and/or Perlon®.

8. Method according at least to one of the claims 1 to 7, **characterised in that** the surfaces are channels surfaces of a microanalysis system.

9. Method according at least to one of the claims 1 to 7, **characterised in that** the surfaces are surfaces of synthetic microparticles.

10. Method according at least to one of the claims 1 to 9, **characterised in that** the cells are provided in a solution which contains:
- saline buffer
- inositol and
- polyvinyl alcohol.

11. Method according to claim 10, **characterised in that** the saline buffer is PBS.

12. Method according to claim 10 or 11, **characterised in that** the proportion of saline buffer and/or inositol is variable.

13. Method according at least to one of the claims 10 to 12, **characterised in that** the content of PVA is between 0.1% (w/v) and 1.1% (w/v), preferably between 0.3% (w/v) and 0.6% (w/v), particularly preferred between 0.45% (w/v) and 0.55% (w/v).

14. Use of a solution containing:
- saline buffer
- inositol, and
- polyvinyl alcohol,
the content of polyvinyl alcohol being between 0.1% (w/v) and 1.1% (w/v),
in order to prevent adhesion of cells on surfaces in microanalysis systems.

15. Use according to claim 14, **characterised in that** the saline buffer is PBS.

16. Use according to claim 14 or 15, **characterised in that** the content of polyvinyl alcohol is between 0.3% (w/v) and 0.6% (w/v), preferably between 0.45% (w/v) and 0.55% (w/v).

## Revendications

1. Procédé pour empêcher l'adhérence de cellules à des surfaces, **caractérisé en ce que** les cellules sont préparées dans une solution contenant un tampon salin et de l'alcool polyvinylique.

2. Procédé selon la revendication 1, **caractérisé en ce que** la concentration en alcool polyvinylique en solution atteint 0,01 % (w/v) à 5 % (w/v).

3. Procédé selon la revendication 2, **caractérisé en ce que** la concentration en alcool polyvinylique en solution atteint 0,01 % (w/v) à 1,1 % (w/v).

4. Procédé selon au moins une des revendications 1 à 3, **caractérisé en ce que** les cellules sont des cellules eucaryotes et/ou procaryotes.

5. Procédé selon au moins une des revendications 1 à 4, **caractérisé en ce que** les surfaces sont des cellules, des membranes cellulaires, des composants cellulaires, des surfaces en silicium, en silicone, en polymères organiques et/ou en verre non revêtues ou revêtues de biomolécules.

6. Procédé selon la revendication 5, **caractérisé en ce que** les biomolécules sont des membranes lipidiques, des lipides, des protéines (notamment des anticorps), des ADN, des acides nucléiques, des messagers, de la biotine, du sucre, des glycanes et/ou des glycoprotéines.

7. Procédé selon la revendication 5, **caractérisé en ce que** les polymères organiques sont le PMMA, le polyuréthane, le polycarbonate, le polypropylène, le polystyrène, le polyéthylène, le chlorure de polyvinyle, le Teflon®, le polyacrylique, le Nylon® et/ou le Perlon®.

8. Procédé selon au moins une des revendications 1 à 7, **caractérisé en ce que** les surfaces sont des surfaces de canaux d'un système de microanalyse.

9. Procédé selon au moins une des revendications 1 à 7, **caractérisé en ce que** les surfaces sont des surfaces de microparticules synthétiques.

10. Procédé selon au moins une des revendications 1 à 9, **caractérisé en ce que** les cellules sont préparées dans une solution qui contient :
- un tampon salin
- de l'inositol et
- de l'alcool polyvinylique.

11. Procédé selon la revendication 10, **caractérisé en ce que** le tampon salin est le PBS.

12. Procédé selon la revendication 10 ou 11, **caractérisé en ce que** la proportion de tampon salin et/ou d'inositol est variable.

13. Procédé selon l'une des revendications 10 à 12, **caractérisé en ce que** la teneur en PVA se situe entre 0,1 % (w/v) et 1,1 % (w/v), de préférence entre 0,3 % (w/v) et 0,6 % (w/v), particulièrement préférentiellement entre 0,45 % (w/v) et 0,55 % (w/v).

14. Utilisation d'une solution contenant .
- un tampon salin
- de l'inositol et
- de l'alcool polyvinylique,
la teneur en alcool polyvinylique se situant entre 0,1 % (w/v) et 1,1 % (w/v),
pour empêcher l'adhérence des cellules à des surfaces dans des systèmes de microanalyse.

15. Utilisation selon la revendication 14, **caractérisée en ce que** le tampon salin est le PBS.

16. Utilisation selon la revendication 14 ou 15, **caractérisée en ce que** la teneur en alcool polyvinylique se situe entre 0,3 % (w/v) et 0,6 % (w/v), de préférence entre 0,45 % (w/v) et 0,55 % (w/v).
